Europäisches Patentamt

European Patent Office

Office européen des brevets

(19)

(11) Publication number: **0 396 344**

**A2**

(12) # EUROPEAN PATENT APPLICATION

(21) Application number: **90304599.5**

(22) Date of filing: **27.04.90**

(51) Int. Cl.5: **C12P 19/04, A61L 29/00, A61L 17/00**

(30) Priority: **28.04.89 JP 111077/89**
**27.12.89 JP 344292/89**
**19.01.90 JP 9892/89**

(43) Date of publication of application:
**07.11.90 Bulletin 90/45**

(84) Designated Contracting States:
**DE FR GB**

(71) Applicant: **AJINOMOTO CO., INC.**
**5-8, Kyobashi 1-chome, Chuo-ku**
**Tokyo 104(JP)**

Applicant: **SONY CORPORATION**
**7-35, Kitashinagawa 6-chome Shinagawa-ku**
**Tokyo(JP)**

(72) Inventor: **Yamanaka, Shigeru, c/o Central**
**Research Lab.**
**AJINOMOTO CO. INC., 1-1, Suzuki-cho**

Kawasaki-ku, Kawasaki-shi, Kanagawa(JP)
Inventor: **Ono, Eiju, c/o Central Research Lab.**
**AJINOMOTO CO. INC., 1-1, Suzuki-cho**
**Kawasaki-ku, Kawasaki-shi, Kanagawa(JP)**
Inventor: **Watanabe, Kunihiko c/o Central**
**Research Lab.**
**AJINOMOTO CO. INC., 1-1, Suzuki-cho**
**Kawasaki-ku, Kawasaki-shi, Kanagawa(JP)**
Inventor: **Kusakabe, Masahiro, c/o SONY**
**CORPORATION**
**6-7-35, Kitashinagawa, Shinagawa-ku**
**Tokyo(JP)**
Inventor: **Suzuki, Yoshiaki, c/o SONY**
**CORPORATION**
**6-7-35, Kitashinagawa, Shinagawa-ku**
**Tokyo(JP)**

(74) Representative: **Armitage, Ian Michael et al**
**MEWBURN ELLIS 2 Cursitor Street**
**London EC4A 1BQ(GB)**

(54) Hollow microbial cellulose, process for preparation thereof, and artificial blood vessel formed of said cellulose.

(57) A hollow microbial cellulose comprising a cellulose produced by a microorganism is described. The microbial cellulose may be in the form of a composite comprising the microbial cellulose which is either integrated with another substance or adheres mainly to one or both of the inner and outer surface of a hollow carrier. The microbial cellulose is prepared by culturing a cellulose-producing microorganism on one or both of the inner surface and outer surface of an oxygen-permeable hollow carrier; or impregnating a cellulose produced by a microorganism with a medium, curing the cellulose if necessary, and cutting the cellulose. The microbial cellulose is useful as a substitute for a blood vessel or another internal hollow organ, or another medical material, an immobilizing carrier for enzymes, microorganisms and cells, an industrial material, and a chemical material.

# HOLLOW MICROBIAL CELLULOSE, PROCESS FOR PREPARATION THEREOF. AND ARTIFICIAL BLOOD VESSEL FORMED OF SAID CELLULOSE

The present invention relates to a hollow cellulose composed of a cellulose produced by a microorganism, a process for the preparation of this cellulose and an artificial blood vessel formed of this cellulose.

This hollow microbial cellulose can be used as an immobilizing carrier for various enzymes, microorganisms and cells, a tubular industrial material, a medical material, a chemical material and the like, as well as other synthetic polymers. For example, in the medical field, the hollow microbial cellulose can be used as a substitute for an internal hollow organ such as the ureter, the trachea, a digestive tract, a lymphatic vessel or a blood vessel.

It is known that a cellulose is produced by using a microorganism, especially acetic acid bacteria (a cellulose produced by a microorganism will be referred to as "microbial cellulose" hereinafter), and this cellulose is used as an industrial material, a medical material, a chemical material and the like.

According to the technique disclosed in Japanese Unexamined Patent Publication No. 62-36467, a filmy cellulose produced by acetic acid bacteria is press-dried or disintegrated and is used alone or in the form of a composite with another material as a high-mechanical-strength molding material. Japanese Unexamined Patent Publication No. 62-8709 discloses a process for producing a beady microbial cellulose to be used as an immobilizing carrier or the like, and Japanese Unexamined Patent Publication No. 61-221201 discloses a process for producing ultra-fine particles by acid-hydrolyzing a microbial cellulose. Furthermore, as disclosed in Japanese Unexamined Patent Publication No. 59-120159, Japanese Unexamined Patent Publication No. 63-152601, and Japanese Unexamined Patent Publication No. 01-50815, it is considered that, since this microbial cellulose has a very high compatibility with a living body, the microbial cellulose is extremely useful as a medical pad, an artificial skin, a cultured skin carrier, a carrier for the mass culture of cells and an additive to the interior of the oral cavity.

In the as-prepared state, the microbial cellulose has a net work structure in which very fine ribbon-shaped fibers (it has been stated that the width or diameter is smaller than 100 nm) composed of a highly crystalline and highly uniaxially oriented cellulose are complicatedly entangled with one another, and this network structure contain a large quantity of a liquid in interior voids thereof and has a gel-like appearance. The liquid component contained in the voids, for example, water, is present as free water, and if an external force is applied, the liquid is easily squeezed out. Since the cellulose is composed of many ribbon-shaped fibers having a high crystallinity, the cellulose can resist external forces such as a tensile force even in the wet state. The microbial cellulose is not structurally different from a cellulose originating from a plant, but a high-order structure such as the above-mentioned structure is not found in the plant-originating cellulose although it is characteristic of the microbial cellulose. Accordingly, the microbial cellulose has various characteristics. For example, the microbial cellulose has a high strength though it is gelatinous.

Nevertheless, this microbial cellulose as one polymeric material poses various problems, compared with other widely used polymeric materials. More specifically, since thermoplastic polymeric materials represented by polyethylene and polyesters are made plastic by an application of heat or the addition of a softener, they can be molded into a desirable shape without changing the physical properties thereof. Furthermore, these polymeric materials can be formed in to any required shape or can be laminated by dissolution in a solvent or the like. In contrast, since the microbial collulose has the above-mentioned network structure and has no plasticity, if the microbial cellulose is dissolved in a solvent, the characteristics based on the characteristic high-order structure of the microbial cellulose are lost, and no substantial difference can be found between the microbial cellulose and the plant-originating cellulose.

The as-prepared microbial cellulose appears to be gelatinous; namely, the microbial cellulose is composed of fine fibers as pointed out above, and a liquid component is contained in voids among the fibers in an amount of at least 95% by weight. This liquid component contained in the voids among the fibers in this gelatinous material is not restrained in the molecule state as in usual polymeric gels. For example, where the liquid component is water, the majority of water is present as free water in the gelatinous microbial cellulose, and the liquid component can be squeezed out by lightly pressing the microbial cellulose by hand. Since the microbial cellulose has no plasticity and the contained liquid component is not restrained, it is difficult to directly mold the gelatinous microbial cellulose into a desirable shape or size while utilizing the original structure and characteristics. For example, a long band having a length of several to several hundred meters, a spherical product having a diameter of several centimeters, or a hollow product such as a pipe, cannot be obtained from the microbial cellulose by the conventional technique, and therefore, the use of the microbial cellulose in industrial fields is remarkably restricted with regard to the shape, size, and processability. For example, a certain strength is required when the microbial cellulose is used as a hollow immobilizing carrier or an artificial organ embedded in a living body, such as an artificial ureter, trachea, digestive tact, lymphatic vessel or blood vessel, but this requirement is not satisfied by the microbial cellulose.

In connection with an artificial blood vessel as an artificial organ, a substitute for a large-diameter artery having an inner diameter exceeding about 6 mm can be successfully provided, but a substitute for small-diameter artificial vessels such as small-diameter arteries, veins and capillary vessels, which occupy the majority of blood vessels in the body, have not been developed.

This is because the problem of blocking due to the formation of a thrombus has not been solved. Namely, artificial vessels now clinically used have a simple structure such as a woven or knitted fabric of a polyester fiber or a porous body of polytetrafluoroethylene, and when such an artificial blood vessel is implanted in a living body and a living body texture is formed around the implanted structure, the embedded body acts as an artificial blood vessel for the first time. Therefore, in the case of a small-diameter blood vessel, problems such as a blocking due to the formation of a thrombus often arise before the formation of the pseudo living body structure. The thrombus blocks the flow of blood stream, or is carried by the blood stream to cause cerebral thrombosis, myocardial infraction or pulmorary infraction, causing a fatal crisis to occur in the human body.

As apparent from the above description, in the case of a substitute for a small-diameter blood vessel, a more severe control of the formation of a thrombus is required until a stable living body texture is formed, and to this end, various approaches have been made in many fields. As typical instances there can be mentioned a heparin treatment, a urokinaze-immobilizing treatment, and a plasma treatment.

A primary object of the present invention is to provide a hollow material which is extremely useful as an immobilizing carrier, an industrial material, a medical material including an artificial organ, and a chemical material.

Another object of the present invention is to provide an artificial blood vessel which has a very good compatibility with a living body and can be used as a substitute for a small-diameter blood vessel having an inner diameter smaller than 6 mm.

In accordance with the present invention, there is provided a hollow microbial cellulose comprising a cellulose produced by a microorganism. The hollow microbial cellulose is obtained by culturing a cellulose-producing microorganism on the inner surface and/or outer surface of an oxygen-permeable hollow carrier composed of, for example, cellophane, Teflon, silicon, ceramics, a non-woven fabric or a woven fabric. Furthermore, the hollow microbial cellulose is obtained by impregnating a cellulose produced by a microorganism with a medium, curing the cellulose if necessary, and then cutting the cellulose.

The cellulose produced by a microorganism is characterized as being composed of $\alpha$-cellulose having a high crystallinity, having a very high surface orienting property and a very high strength, and suffering very little degradation thereof in a living body, and exhibiting a very good compatibility with a living body, particularly a compatibility with blood.

The cellulose produced by a microorganism comprises a cellulose, heteropolysaccharides comprising a cellulose as the main chain, and $\beta$- and $\alpha$-glucans. In addition to the cellulose, the heteropolysaccharides comprise, as constituents, hexoses such as mannose, fructose, galactose, and rhamnose, pentoses such as xylose and arabinose, and organic acids such as uronic acid. One of the foregoing polysaccharides is present alone in the cellulose, or a plurality of the polysaccharides are present in the mingled state in the cellulose.

In the present invention, the kind of cellulose-producing microorganism used is not particularly critical. For example, there can be mentioned Acetobacter pasturianus, ATCC 10821, Acetobactor aceti, Acetobacter ransens, Sarcina ventriculi, Bacterium xyloides bacteria belonging to the genus Pseudomonas, bacteria belonging to the genus Agrobacterium, and bacteria belonging to Rhizobium.

The cellulose is formed and accumulated by a usual bacterium-culturing method using a microorganism as mentioned above. Namely, the microorganism is added to a usual nutrient broth comprising a carbon source, a nitrogen source, inorganic salts and, if necessary, organic micronutrients such as amino acids and vitamins, and the culturing is conducted at a temperature of 20 to 40 $^{\circ}$C.

The first process for preparing the hollow microbial process comprises culturing a cellulose-producing microorganism on the inner surface and/or outer surface of an oxygen-permeable hollow article, for example, a hollow body composed of cellophane, Teflon, silicon, ceramics, a non-woven fabric or a woven fabric. More specifically, an oxygen-permeable article having a hollow shape (hereinafter referred to as "hollow carrier"), for example, a hollow carrier formed of cellophane, Teflon, silicon, ceramics, a non-woven fabric or a woven fabric, is immersed in a culture liquid, a cellulose-producing microorganism and a culture medium are supplied to the inner side and/or outer side of the hollow carrier, and the culturing is carried out by introducing an oxygen-containing gas or liquid to the inner side and/or outer side of the hollow carrier. In the case of a hydrophilic hollow carrier, if a culture liquid containing the microorganism need be applied only to the inner surface and/or outer surface of the hollow carrier. If the culturing is conducted in this manner, a gelatinous cellulose having a thickness of 0.01 to 20 mm is formed on the surface of the carrier. There is a certain mutual action between the cellulose-producing microorganism and/or produced cellulose and the hollow carrier, and where the microorganism and/or cellulose adheres to or falls into contact with the carrier, a composite of the cellulose and hollow carrier is finally formed. If holes allowing the passage of the cellulose or microorganism, i.e., holes having a diameter of 0.1 $\mu$m to 5 mm, are formed, sometimes a cellulose formed on one surface of the hollow carrier will be connected to a cellulose formed on the opposite surface, and accordingly, a composite comprising a microbial cellulose adhering to the inner surface and/or outer surface of the hollow carrier is obtained. Where the cellulose is not bonded to the carrier, if the hollow carrier is removed after the preparation of the cellulose, a hollow shaped article composed solely of the cellulose can be obtained.

Since the thus-prepared cellulose contains cells of the microorganism or culture medium ingredients, the cellulose can be washed as required, and this washing is carried out by using a dilute alkali, a dilute acid, an organic solvent and hot water, alone or in any combination thereof.

In addition to the above-mentioned composite comprising the cellulose adhering to the hollow carrier, another composite can be prepared by integrally combining the hollow cellulose with a substance other than the microbial cellulose as an auxiliary material. As the auxiliary material, there can be mentioned granules and fibers of inorganic and organic materials such as alumina, glass and crystalline cellulose, and water-soluble, polar solvent-soluble or hydrophilic gel-forming polymeric materials such as agar, dextran, polyacrylamide, polyvinyl pyrrolidone, alginic acid salts, hyaluronic acid, curdlan, polyacrylic acid salts, heparin, sulfated polysaccharides, pullulan, carrageenan, glucomannan, cellulose derivatives, polyethylene glycol, polyvinyl alcohol, gelatin, collagen, laminitol, fibronectin, keratin, silk hydrolyzate, polyamino acids, poly-organic acids and enzymes. The hollow microbial cellulose is combined with an auxiliary material as mentioned above by such means as impregnation, lamination or adsorption to obtain a composite. Furthermore, a composite comprising a granular or gelatinous auxiliary material included in the three-dimensional structure of the microbial cellulose, and a composite comprising a fibrous auxiliary material entangled with the texture of the cellulose can be obtained.

The second process for obtaining the hollow microbial cellulose comprises impregnating a cellulose produced by a microorganism with a mediun, curing the cellulose if necessary, and cutting the cellulose. More specifically, a microorganism is inoculated in a vessel charged with a culture medium as mentioned above and stationary culturing is carried out to grow a gelatinous microbial cellulose on the surface of the culture medium. This microbial cellulose is impregnated with a medium, and if necessary, curing, freezing and thickening are carried out. Accordingly, the liquid component is restrained among fibers constituting the microbial cellulose, to inhibit a free movement of the liquid component, and then the cutting operation is carried out.

As the medium, there can be used polyhydric alcohols such as glycerol, erythritol, glycol, sorbitol and maltitol, saccharides such as glucose, galactose, mannose, maltose and lactose, natural and synthetic high polymeric substances such as polyvinyl alcohol, polyvinyl pyrrolidone, polyethylene glycol, carboxymethyl cellulose, agar, starch, alginic acid salts, xanthane gum, polysaccharides, oligosaccharides, collagen, gelatin, and proteins, and water-soluble polar solvents such as acetonitrile, dioxane, acetic acid, and propionic acid. These media can be used alone or in the form of a mixture or two or more thereof. Furthermore, a solution containing an appropriate solute can be used as the medium.

After the microbial cellulose is impregnated with the medium, the impregnated medium is preferably frozen by cooling, or a chemical reaction, for example, crosslinking, is caused to cure the impregnated medium or increase the viscosity, whereby the liquid component is restrained among fibers of the microbial cellulose. By thus restraining the liquid component, it becomes possible to easily cut the gelatinous microbial cellulose by a usual machine such as a drill.

A series of the foregoing treatments will now be specifically described with reference to an embodiment using glycerol.

A gelatinous microbial cellulose obtained by the stationary culture is immersed in an aqueous solution of glycerol having a concentration of 0.5 to 40% by weight, and the cellulose is frozen at -80°C. The cellulose in the frozen state is bored by using a cork borer or other tool or is cut by a knife or the like, to obtain a shaped body having a required shape. The temperature is then again raised to room temperature and the shaped body is immersed in an appropriate solvent such as water or a physiological saline solution, to remove glycerol present among fibers, whereby a hollow microbial cellulose shaped body is obtained.

If the thus-prepared cellulose is dried, fine ribbon-shaped fibers constituting the gelatinous cellulose are glued to one another through hydrogen bonds, and therefore, a rigid filmy or plastic product is obtained. The hardness can be varied by adjusting the quantity of hydrogen bonds originating from hydroxyl groups of the cellulose at the drying by adding a softener such as glycerol. Furthermore, if the drying is effected by a means not causing hydrogen bonds, such as freeze drying, critical point drying or drying after solvent substitution, a porous product different from the above-mentioned rigid film can be prepared.

The third process for preparing a hollow microbial cellulose shaped article comprises preparing two glass tubes differing in the diameter, inserting the small-diameter glass tube into the large-diameter glass tube, culturing a microorganism in a cylindrical space between the two glass tubes to form a microbial cellulose having a cylindrical shape, and withdrawing the cylindrical cellulose product.

If the prepared hollow microbial cellulose shaped article and composite are subjected to a shaping treatment such as a bellows-forming treatment, shaped articles having greatly improved handling property and softness can be formed.

Since the thus-prepared hollow microbial cellulose shaped article and composite can be formed into various products differing in shape, as pointed out above, they can be used as immobilizing carriers for enzymes, cells, bacteria and the like, hollow industrial materials, medical materials, chemical materials, and the like, as well as other synthetic polymeric materials.

When the hollow microbial cellulose is used as an artificial blood vessel, the as-prepared hollow microbial cellulose can be directly substituted for a blood vessel in a living body, or the hollow microbial cellulose can be subjected to a certain preliminary treatment. For example, an adhesion of endothelial cells to the surface of the hollow microbial cellulose can be mentioned as the preliminary treatment.

The hollow microbial cellulose has a very good compatibility with a living body, especially blood, and has a high surface orienting property and a high mechanical strength. When this microbial cellulose is used as an artificial blood vessel material, the formation of a thrombus is controlled and the microbial cellulose can be satisfactorily substituted for a small-diameter blood vessel having an inner diameter smaller than 6 mm. The present invention will now be described in detail with reference to the following examples, in which all of "%" are by weight.

## Example 1

A culture medium comprising 5 g/dl of sucrose, 0.5 g/dl of a yeast extract (supplied by Difco), 0.5 g/dl of ammonium sulfate, 0.3 g/dl of monopotassium phosphate, and 0.5 g/dl of magnesium sulfate heptahydrate and having a pH value of 5.0 was maintained at 120°C for 20 minutes in an autoclave, and the culture medium was inoculated with Acetobacter acet subspecies xylinum (ATCC 10821) at a concentration of $1 \times 40^4$ cells per ml. The liquid was charged in a dialysis tube of cellophane having an inner diameter of 5 mm and a length of 15 cm, the tube was sealed by binding both the ends of the tube, and culturing was conducted at 30°C in air for 3 weeks, whereby a gelatinous microbial cellulose having a thickness of about 2 mm was produced on the inner surface of the dialysis tube. The cellulose was recovered and washed with a 2% solution of sodium hydroxide to remove the cells and culture medium ingredients, and the cellulose then washed with an excessive amount of water to obtain a hollow gelatinous microbial cellulose.

## Example 2

A hollow gelatinous microbial cellulose was obtained in the same manner as described in Example 1 except that a silicon tube having an inner diameter of 3 mm, an outer diameter of 4 mm, and a length of 30 cm was used.

## Example 3

A silicon tube having an inner diameter of 2 mm, an outer diameter of 3 mm, and a length of 10 cm was placed in the inoculated culture mediun described in Example 1, and air was introduced at a rate of 10 ml/min while applying a pressure of atmospheric pressure plus 0.1 atmosphere to the interior of the silicon tube. After 2 weeks, a microbial cellulose produced around the tube was recovered and washed to obtain a hollow microbial cellulose.

## Example 4

A cylindrical cotton fabric having an inner diameter of 3 mm, a length of 10 cm, and a thickness of about 0.5 mm was charged in a vessel filled with the inoculated culture medium described in Example 1, to a depth of 30 cm, and humidified germ-free air was introduced into the interior of the cylindrical fabric at a rate of 50 ml/min in such a manner that air did not leak from the surface of the cylindrical fabric. After 2 weeks, a gelatinous microbial cellulose having a thickness of about 1 to about 2 mm was produced on the inner and outer walls of the fabric. The cellulose was immersed in a 4% solution of NaOH at 70°C for 3 hours to remove the cells and culture medium ingredients, whereby a composite comprising the microbial cellulose integrated with the cotton fabric was obtained.

Example 5

The microbial cellulose/hollow fabric composite obtained in Example 4 was immersed in a 0.5% solution of glycerol at room temperature for 5 hours, and the composite then dried to obtain a dry product of a cotton fabric/microbial cellulose composite.

Example 6

A hollow cotton fabric having an inner diameter of 3 mm, a length of 10 cm, and a thickness of about 0.5 mm was charged in a vessel filled with the inoculated culture medium described in Example 1, to a depth of 30 cm, and Florinate FC-40 (supplied by 3M) saturated with humidified air was introduced into the interior of the hollow fabric at a linear speed of 3 cm/min. After 2 weeks, a gelatinous microbial cell having a thickness of about 1 to about 2 mm was produced on the inner and outer walls of the fabric. The fabric was immersed in a 4% solution of NaOH at 70°C for 3 hours to remove the cells and culture medium ingredients, and thus a composite comprising the microbial cellulose integrated with the cotton fabric was obtained.

Example 7

The culture mediun described in Example 1 was inoculated with Acetobacter acet subspecies xylinum (ATCC 10821) at a concentration of 1 x 40⁴ cells per ml, the liquid was charged into a double cylindrical test tube which had been autoclaved, and culturing was conducted at 30°C in air for 2 months. A gelatinous hollow microbial cellulose having a thickness of about 8 cm was formed on the surface of the culture liquid. The cellulose was recovered and boiled for 1 hour in a 2% solution of sodium hydroxide of a volume 10 times the volume of the cellulose. This boiling operation was repeated three times, whereby the cells and culture medium ingredients were removed. After the boiling, the hollow microbial cellulose was washed with an excessive quantity of water, until the pH value became neutral, and the washed cellulose was dried at 105°C for 4 hours to obtain a hollow microbial cellulose having a diameter of 1 mm.

Example 8

A vessel having a size of 30 cm x 30 cm x 20 cm (dopth), which had been autoclaved, was charged with 10 ℓ of a culture medium prepared in the same manner as described in Example 7, and culturing was conducted at 30°C in air for 50 days. A filmy cellulose having a thickness of about 3 cm was produced on the surface of the culture liquid. The collulose was recovered and then boiled for 1 hour in a 2% solution of sodium hydroxide of a volume 10 times the volume of the cellulose, to remove the cells and culture medium ingredients, and the boiled filmy cellulose was washed with an excessive quantity of water, until the pH value became neutral. The filmy cellulose was immersed in a 10% solution of glycerol for 10 hours and maintained at -80°C for 10 hours. The filmy cellulose was formed into an article having a length of 20 cm, an inner diamerer of 2 mm, and an outer diameter of 8 mm by using a cork borer having a special thin blade. The article was boiled in distilled water for 30 minutes and dried at 105°C for 4 hours. Then the article was autoclaved at 120°C for 30 minutes and immersed in a heparin solution having a concentration of 10 μg/ml a whole day and night, and thereafter the article was sterilely dried to obtain a hollow microbial cellulose composite.

Example 9

By using the culture medium described in Example 1 and the double cylindrical test tube described in Example 7, Acetobacter pasturianus, ATCC 23769, was inoculated and cultured to obtain a gelatinous hollow microbial cellulose having a length of about 7 cm and having a shape similar to that of the gelatinous hollow microbial cellulose obtained in Example 7. Then the cellulose was washed and treated in the same manner as described in Example 7 to obtain a hollow microbial cellulose having a diameter of 1.2 mm.

Example 10

An Erlenmeyer flask having an inner capacity of 200 ml was charged with 50 ml of a culture medium comprising 5 g/dl of sucrose, 0.5 g/dl of yeast extract, 0.5 g/dl of ammonium sulfate, 0.3 g/dl of potassium hydrogenphosphate, and 0.05 g/dl of magnesium sulfate, and having a pH value of 5.0, and the culture medium was steam-sterilized at 120°C for 20 minutes to form a culture liquid.

Then the culture liquid was poured into a cylindrical space of a double glass tube, the culture liquid was inoculated with Acetobacter aceti subspecies xylynum (ATCC 10821) which had been grown at 30°C for 3 days in a test tube slant agar culture medium comprising 0.5 g/dl of yeast extract, 0.3 g/dl of peptone and 2.5 g/dl of mannitol, and having a pH value of 6, and culturing was carried out at 30°C for 30 days, whereby a white microbial cellulose was densely formed in a space of the double glass tube.

The double glass tube was dismantled, and the cylindrical microbial cellulose was taken out and thoroughly washed sufficiently with water to obtain an artificial blood vessel having an inner diameter of about 2 to about 3 mm.

The blood compatibility (antithrombic property) was evaluated by the blood vessel substitution test using an adult mongrel dog. More specifically, parts of the descending aorta and jugular vein of the adult mongrel dog were substituted with the prepared artificial blood vessel, and after 1 month, the artificial blood vessel was taken out and the state of the adhesion of thrombi was examined. A slight adhesion of thrombi was observed in the sutured portion, but substantial adhesion of thrombi was observed on the inner surface of the blood vessel, and a good open state was maintained.

As apparent from the above description the hollow microbial cellulose of the present invention is very useful as an immobilizing carrier, an industrial material, a medical material including an artificial organ, a chemical material and the like. Moreover, the hollow microbial cellulose of the present invention has a particularly good compatibility with blood and a superior durability and uniformity. Accordingly, an artificial blood vessel that can be used for a small-diameter blood vessel having a small inner diameter, e.g., smaller than 6 mm, can be provided according to the present invention.

**Claims**

1. A hollow microbial cellulose comprising a cellulose produced by a microorganism.

2. A hollow microbial cellulose as set forth in claim 1, which is in the form of a composite comprising the hollow microbial cellulose adhering mainly to one or both of the inner surface and outer surface of a hollow carrier.

3. A hollow microbial cellulose as set forth in claim 1, which is in the form of a composite comprising the hollow microbial cellulose integrated with another material.

4. A process for the preparation of a hollow microbial cellulose, which comprises culturing a cellulose-producing micrcorganism on one or both of the inner surface and outer surface of an oxygen-permeable hollow carrier.

5. A process according to claim 4, wherein the oxygen-permeable hollow carrier is composed of at least one member selected from the group consisting of cellophane, Teflon, silicon, ceramics, nonwoven fabrics, and woven fabrics.

6. A process for the preparation of a hollow microbial cellulose, which comprises impregnating a cellulose produced by a microorganism with a medium, curing the cellulose if necessary, and cutting the cellulose.

7. A process according to claim 6, wherein the medium is at least one member selected from the group consisting of polyhydric alcohols, polysaccharides, proteins, natural high polymeric substances, and synthetic high polymeric substances.

8. A process according to claim 6, wherein the medium is glycerol.

9. An artificial blood vessel composed mainly of a cellulose produced by a microorganism.